# FASCICULE DE BREVET EUROPEEN

(11) **EP 2 900 323 B1**
(45) Date de publication et mention de la délivrance du brevet: **09.11.2016**
(21) Numéro de dépôt: 13771097.6
(22) Date de dépôt: 26.09.2013
(51) Int. Cl.: A61N 5/10

(54) **PROCÉDÉ D'ÉVALUATION D'UNE MÉTHODE DE DÉTERMINATION DE LA DOSE CUMULÉE REÇUE AU COURS D'UN TRAITEMENT DE RADIOTHÉRAPIE**
VERFAHREN ZUR BEWERTUNG EINES VERFAHRENS ZUR BESTIMMUNG DER WÄHREND EINER STRAHLENBEHANDLUNG EMPFANGENEN AGGREGATDOSIS
METHOD OF EVALUATING A PROCEDURE FOR DETERMINING THE AGGREGATE DOSE RECEIVED IN THE COURSE OF A RADIOTHERAPY TREATMENT

(30) Priorité: 26.09.2012 FR 1259054
(43) Date de publication de la demande: 05.08.2015
(73) Titulaire: Université de Rennes I, 35000 Rennes Cedex (FR)
(72) Inventeur: DE CREVOISIER, Renaud, F-35700 Rennes (FR); HAIGRON, Pascal, F-35700 Rennes (FR); SIMON, Antoine, F-35700 Rennes (FR); CAZOULAT, Guillaume, F-35700 Rennes (FR); DUMENIL, Aurélien, F-35000 Rennes (FR)
(74) Mandataire: Vidon Brevets & Stratégie
(86) Numéro de dépôt international: PCT/EP2013/070027
(87) Numéro de publication internationale: WO 2014/049030

(56) Documents cités:
- WO-A1-2012/123894
- MIYABE YUKI ET AL: "New algorithm to simulate organ movement and deformation for four-dimensional dose calculation based on a three-dimensional CT and fluoroscopy of the thorax", MEDICAL PHYSICS, vol. 36, no. 10, 4 septembre 2009 (2009-09-04), pages 4328-4339, XP012129704, AIP, MELVILLE, NY, US ISSN: 0094-2405, DOI: 10.1118/1.3213083
- WEN NING ET AL: "Evaluation of the deformation and corresponding dosimetric implications in prostate cancer treatment.", PHYSICS IN MEDICINE AND BIOLOGY, vol. 57, no. 17, 7 septembre 2012 (2012-09-07), pages 5361-5379, XP002693995, ISSN: 1361-6560

## Description

### 1. Domaine de l'invention

Le domaine de l'invention est celui de la radiothérapie, et en particulier de la détermination des doses de rayonnement reçues par des organes, en cours de radiothérapie.

Plus précisément, l'invention concerne le contrôle de la qualité d'une méthode de calcul d'une dose cumulée mise en oeuvre par un système de radiothérapie prédéterminé.

### 2. Art antérieur et inconvénients

Comme on le sait, il existe plusieurs types de traitement des cancers, parmi lesquels la radiothérapie occupe une place essentielle. Ainsi, en France, on diagnostique environ 280 000 cancers par an, dont environ 200 000 sont traités, au moins en partie, par radiothérapie.

La radiothérapie externe, à laquelle s'applique l'invention, met en oeuvre un accélérateur linéaire placé à environ un mètre du patient, générant des faisceaux d'irradiation (de photons, d'électrons ou de protons, selon les techniques mises en oeuvre) convergeant vers la tumeur et délivrant une dose d'irradiation relativement homogène dans la zone d'intersection des faisceaux.

La radiothérapie met en oeuvre une irradiation, potentiellement dangereuse, et qui doit donc être précisément contrôlée, tant en ce qui concerne la dose cumulée reçue par l'organe à soigner que les doses reçues, inévitablement, par les organes sains voisins (qui doit bien sûr être minimisée).

Un traitement par radiothérapie repose notamment sur une phase préalable de planification au cours de laquelle la balistique d'irradiation (les paramètres de l'accélérateur linéaire) est déterminée. La dose qui sera théoriquement reçue par les organes est alors calculée. Cette étape repose sur l'acquisition d'une image scanner permettant de visualiser la cible tumorale et les organes à risque. Le traitement est donc planifié suivant une configuration des organes (en termes de taille et de forme) fixe.

Cependant, durant le traitement, qui est fractionné en séances (ex. : 40 séances pour le cancer de la prostate), ces organes se déforment (ex. : vessie, rectum pour le cancer de la prostate). La dose qu'ils reçoivent est alors différente de la dose planifiée.

Afin d'estimer la dose cumulée réellement reçue par les organes lors des différentes fractions (séances) de traitement, des méthodes reposant sur l'estimation des déformations des organes sont proposées. Celles-ci reposent sur une imagerie embarquée (par exemple de type CBCT) permettant de visualiser les organes lors de chaque fraction. Des méthodes de recalage d'image (appliquées entre les CBCT et le scanner de planification) permettent l'estimation des déformations, qui sont ensuite appliquées à la dose journalière, de façon à propager sur le scanner de planification la dose délivrée et à pouvoir ainsi la cumuler.

Différentes méthodes de calcul de la dose cumulée sont proposées, mais le contrôle qualité de ces méthodes reste non résolu. En effet, il s'agit de méthodes théoriques, et il n'existe pas de moyens pour mesurer, *in situ,* les doses réellement reçues par chaque organe.

En effet, ces méthodes reposent sur une étape de recalage d'images qui fournit une estimation de la déformation des organes entre les fractions de traitement. Cette estimation introduit des imprécisions locales, qui sont d'amplitude et de localisation variables suivant l'algorithme et les images considérés. Or cette variabilité d'amplitude et de localisation des imprécisions de recalage a un impact important sur la qualité de la dose cumulée estimée. Par exemple, une imprécision de recalage localisée sur une région anatomique associée à une dose uniforme entraînera une erreur de dose cumulée très limitée comparée à une imprécision de recalage localisée dans une zone de gradient de dose élevé.

Au delà de la précision des champs de déformation, il est fondamental d'évaluer les méthodes de calcul de la dose cumulée en termes dosimétriques.

### 3. Objectifs de l'invention

L'invention a notamment pour objectif de pallier ces inconvénients de l'art antérieur.

Plus précisément, un objectif de l'invention est de fournir une technique permettant d'évaluer, objectivement, la qualité et/ou la précision d'une méthode de calcul de doses cumulées d'irradiation, mise en oeuvre par un système de radiothérapie.

Ainsi, l'invention n'a pas pour objectif de fournir une nouvelle méthode de calcul de doses cumulées, mais de fournir un outil permettant d'évaluer (et le cas échéant améliorer) les méthodes de calcul existantes, ainsi que les futures méthodes qui seront proposées.

L'invention a également pour objectif de fournir une telle technique d'évaluation, qui soit adaptable à de nombreux organes, et en particulier aux organes déformables.

### 4. Exposé de l'invention

Ces objectifs, ainsi que d'autres qui apparaîtront plus clairement par la suite sont atteints à l'aide d'un procédé d'évaluation d'une méthode de détermination de la dose cumulée reçue au cours d'un traitement de radiothérapie, dite méthode à évaluer.

Selon l'invention :
- on définit un modèle déformable d'au moins un organe à traiter et d'au moins un organe voisin, et on génère à l'aide dudit modèle une série d'images simulées, interprétables par la méthode à évaluer;
- on compare la dose cumulée calculée par la méthode à évaluer, appliquée à au moins une desdites images simulées, à une dose cumulée de référence, de façon à délivrer au moins une information représentative de la qualité de ladite méthode,
ladite dose cumulée de référence étant obtenue en tenant compte de déformations anatomiques appliquées auxdits organes, de façon à déterminer une dose de référence effectivement reçue à chaque séance de traitement par lesdits organes, en fonction de leur forme et/ou de leur position réelles lors de cette séance.

Ainsi, l'invention propose une approche efficace pour estimer une dose cumulée de référence, tenant compte des déformations des organes entre les fractions de traitement. On dispose en effet, en tenant compte de ces déformations, d'une estimation précise de la dose réelle qui serait reçue par ce modèle. Ensuite, en considérant les données issues de ce modèle comme données d'entrée de la méthode à évaluer, on dispose de la dose cumulée déterminée par cette dernière. On peut alors comparer cette dose cumulée à la dose cumulée de référence, et en conséquence évaluer la qualité de la méthode à évaluer, de façon objective et précise.

Selon un mode de réalisation particulier de l'invention, l'obtention de ladite dose cumulée de référence met en oeuvre les étapes suivantes :
- génération d'un modèle comprenant au moins un organe à traiter et au moins un organe voisin ;
- détermination d'un champ de déformation desdits organes, à partir d'au moins une règle de déformation et/ou d'évolution desdits organes ;
- obtention d'une série d'images dudit modèle à différents instants, dites images simulées, comprenant au moins une image représentative d'un scanner de planification et une série d'images de suivi, correspondant chacune à une séance de traitement ;
- planification d'un traitement, à partir d'au moins une desdites images simulées, à l'aide d'un système de planification de traitement mettant en oeuvre ladite méthode de détermination de doses de radiothérapie, de façon à obtenir une dose planifiée et des paramètres d'une balistique d'irradiation ;
- détermination d'un ensemble de doses par fraction, correspondant chacune à une desdites séances ;
- détermination de ladite dose cumulée de référence, en appliquant ledit champ de déformation à chaque image de suivi, de façon à déterminer ladite dose de référence effectivement reçue.

Ladite étape de génération d'un modèle peut notamment délivrer un modèle en éléments finis sous la forme d'un maillage en trois dimensions.

Ladite étape de détermination d'un champ de déformations peut en particulier mettre en oeuvre des déformations représentatives de l'élasticité de chaque organe, définies par une loi de comportement de matériaux, par exemple un modèle hyper-élastique.

De plus, lesdites déformations tiennent avantageusement compte des contacts possibles entre lesdits organes.

Ladite étape d'obtention d'une série d'images simulées peut notamment associer, pour chaque image simulée, une vue dudit modèle et une image, éventuellement simplifiée, d'un patient réel, afin de prendre en compte au moins la structure osseuse de ce dernier.

Selon une mise en oeuvre de l'invention, lors de ladite étape de détermination de ladite dose cumulée de référence, la dose associée à chaque image de suivi considérée est déformée suivant le champ de déformations simulé, de façon à propager ladite dose vers l'espace du scanner de référence, et l'ensemble des doses propagées sont sommées pour obtenir la dose cumulée de référence.

La ou lesdites informations représentatives de la qualité de ladite méthode à évaluer peuvent notamment appartenir au groupe comprenant :
- une erreur d'estimation de la dose cumulée en chaque point du champ de déformations, par exemple sous la forme d'une représentation tridimensionnelle des erreurs ;
- des mesures statistiques sur les erreurs d'estimation de la dose cumulée, telles que des erreurs moyenne, minimale et maximale, des écart-type ;
- une représentation des doses cumulées estimée et de référence sous la forme d'histogrammes dose-volume ;
- des mesures statistiques issues d'histogrammes dose-volume, telles que des différences moyenne, minimale, maximale, des sommes des différences ;
- des différences en termes d'indices d'évaluation d'un traitement, telles qu'une dose moyenne, une dose équivalente uniforme, des valeurs prédictives de contrôle tumoral ou de toxicité.

Dans un mode de réalisation particulier de l'invention, ledit organe à traiter appartient au groupe comprenant la prostate, le rectum, la vessie et le canal anal..

L'invention concerne également un produit programme d'ordinateur téléchargeable depuis un réseau de communication et/ou stocké sur un support lisible par ordinateur et/ou exécutable par un microprocesseur, comprenant des instructions de code de programme pour l'exécution du procédé d'évaluation tel que décrit ci-dessus et ci-après, lorsqu'il est exécuté sur un ordinateur.

### 5. Liste des figures

D'autres caractéristiques et avantages de l'invention apparaîtront plus clairement à la lecture de la description suivante de modes de réalisation de l'invention, donnée à titre illustratif et non limitatif et accompagnée des dessins, parmi lesquels :
- la figure 1 est un schéma synoptique illustrant de façon simplifiée la détermination d'une dose cumulée de référence, selon un mode de réalisation de l'invention ;
- les figures 2A et 2B est un exemple de modèle mis en oeuvre selon l'invention, représentatif du pelvis, dans un état initial avant déformation (figure 2A) et après un exemple de déformation (figure 2B) ;
- la figure 3 est un exemple d'insertion d'une image issue du modèle de la figure 2A dans des données d'un patient ;
- la figure 4 est un synoptique illustrant de façon simplifiée l'évaluation d'une méthode à évaluer, utilisant une dose cumulée de référence déterminée selon l'approche de la figure 1 ;
- la figure 5 est un exemple de comparaison d'histogrammes dose-volume obtenu selon l'invention.

### 6. Description d'un mode de réalisation de l'invention

### 6.0 introduction

L'invention propose donc un procédé d'évaluation, permettant notamment le contrôle qualité du calcul de la dose cumulée en radiothérapie déterminée par une méthode de détermination de doses à évaluer (qu'il s'agisse d'une méthode connue, par exemple mise en oeuvre dans des systèmes actuels, ou d'une méthode future).

La solution repose sur une génération de données de simulation numérique basée sur une modélisation mécanique du comportement des organes. Un modèle réaliste de type éléments finis est déformé suivant les caractéristiques mécaniques des organes. Cette approche permet de connaître précisément les correspondances locales des organes, et donc d'obtenir une dose cumulée de référence. Appliquer les méthodes de calcul de la dose cumulée à ces données simulées permet donc de fournir, pour chaque méthode, des mesures évaluant la qualité de son résultat.

L'approche de l'invention repose donc sur deux phases :
- La génération d'un modèle, ou fantôme, numérique correspondant à la région anatomique considérée et le calcul de la dose cumulée de référence sur ce fantôme ;
- L'évaluation des méthodes de calcul de la dose cumulée par l'utilisation du fantôme numérique comme référence.

Le modèle s'appuie sur la génération de données synthétiques issues d'une simulation numérique FEM et fondées sur une modélisation mécanique du comportement des organes. Un modèle éléments finis de forme réaliste est généré et déformé suivant les caractéristiques mécaniques des organes. Un certain nombre de configurations anatomiques sont ainsi obtenues. Cette approche permet de connaître précisément les correspondances locales des organes.

Les maillages obtenus sont ensuite insérés dans des images tridimensionnelles pour obtenir des images simulées correspondant, d'une part, à l'image de planification, et d'autre part, aux images de suivi.

Sur l'image de planification, une optimisation de la balistique est réalisée, fournissant une matrice tridimensionnelle de dose, correspondant à la dose planifiée. De plus, la dose pour chaque image de suivi, ou matrice de dose journalière, est calculée suivant la balistique planifiée. Suivant les matrices de dose journalières et les déformations connues des organes, la dose cumulée de référence est calculée.

### 6.1 Génération du modèle et calcul de la dose cumulée de référence

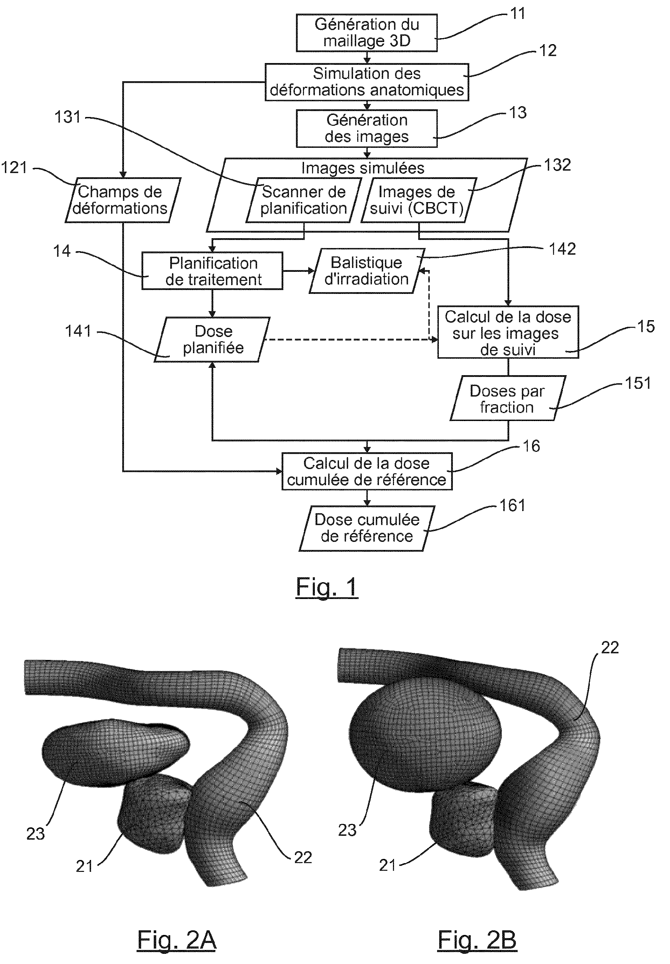

L'invention met en oeuvre, selon le mode de réalisation décrit ci-après, une génération de données synthétiques issues d'une simulation numérique par éléments finis et fondées sur une modélisation mécanique du comportement des organes. Les différentes étapes de la génération des données simulées sont représentées par la figure 1 et décrites ci-dessous.

### 6.1.1 Génération du maillage 3D (11)

Un modèle en éléments finis des organes considérés est généré initialement. Celui-ci représente l'organe cible et les organes à risque environnants (par exemple la prostate 21, le rectum 22, la vessie 23 pour le pelvis, comme illustré par les figures 2A et 2B).

La géométrie des structures anatomiques de ces différents organes est définie soit en considérant des volumes et des formes typiques, soit directement à partir d'images acquises sur un ou plusieurs patients. Ces géométries sont représentées par des surfaces et/ou des volumes. Suivant la géométrie des organes, elles peuvent soit être générées directement en considérant des B-splines modélisant les contours des organes (ex. : rectum), soit en réalisant une forme géométrique qui est discrétisée en surface (ex.: vessie) ou en volume (ex. : prostate), suivant la nature des organes (organes creux ou pleins).

### 6.1.2 Simulation des déformations anatomiques (12)

La simulation des déformations est réalisée, selon ce mode de réalisation, en considérant des propriétés des matériaux classiques (issues de la littérature et/ou de mesures), en terme par exemple de module de Young et de coefficient de Poisson. De même, les épaisseurs des parois des organes creux (ex. : vessie, rectum), ainsi que leur pression interne initiale sont fixées à des valeurs classiques.

Une loi linéaire élastique est utilisée pour décrire le comportement de la prostate et des vésicules séminales, avec un module d'Young de 60 kPa et un coefficient de Poisson de 0.495. Un modèle hyperélastique d'Ogden est défini pour le rectum (et le colon sigmoïde) et la vessie, comme illustré sur un exemple dans le tableau suivant, pour le cas du pelvis.

| Organe | Module d'Ogden µ | Coefficient exponentiel α | Epaisseur de paroi (mm) | Pression |
|---|---|---|---|---|
| Rectum | 0.0424 | 14.598 | 2.28 | 0 à 2 kPa |
| Vessie | 0.0412 | 6.767 | 2.9 | 0 à 5 kPa |

Des conditions aux limites sont également définies, par exemple en fixant les extrémités des organes (rectum et apex de la prostate) et/ou en définissant des liens élastiques (pour représenter les interactions des organes considérés avec leur environnement). De plus, les contacts entre les organes d'intérêt peuvent être définis.

Les déformations des organes sont ainsi simulées suivant les comportements mécaniques modélisés. Par exemple, dans le cas du pelvis, différentes valeurs de pression interne du rectum et de la vessie sont appliquées pour déformer les structures avec une amplitude comparable à celle observée sur patient entre différentes fractions de traitement. Un exemple de résultat de simulation des déformations est représenté figure 2B. On constate, sur cette figure 2B, que les différents organes peuvent se déformer et se déplacer de façon importante, et qu'il est en conséquence essentiel de tenir compte de ce paramètre pour connaître la dose cumulée réellement reçue par chaque organe.

Suivant cette procédure, un champ de déformations 121, correspondant au déplacement de chaque élément du modèle, est connu.

### 6.1.3 Génération des images

Pour chaque configuration des organes considérée dans la modélisation, une image est simulée. Celle-ci correspond soit à une image scanner 131 (pour représenter le scanner utilisé lors de la planification ou une image de suivi, par exemple dans le cas de l'ORL), soit à une image 132, effectuée en cours de traitement, permettant une visualisation des tissus mous en cours d'irradiation. Selon le mode de réalisation illustré, il s'agit d'images CBCT 132 (pour « Cone Beam Computed Tomography » en anglais), pour représenter, par exemple, une image de suivi dans le cas du pelvis. D'autres types d'images 132 peuvent être utilisées, et par exemple des images de tomographie.

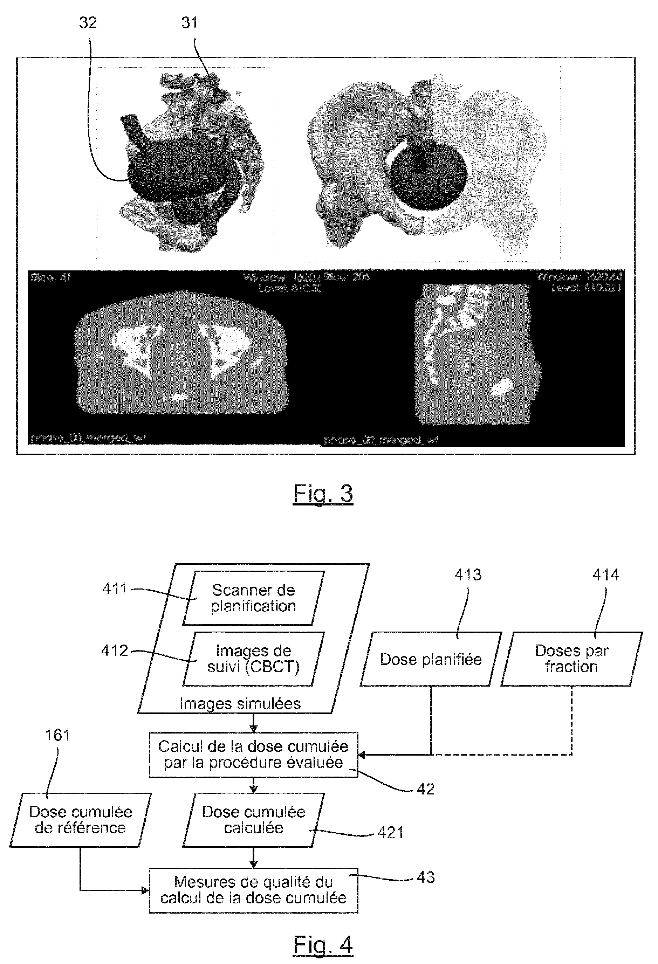

Pour ce faire, comme illustré sur l'exemple de la figure 3, une image scanner ou CBCT 31 d'un patient réel est choisie et simplifiée (par exemple seuls le contour externe et les structures osseuses sont conservées) et les modèles géométriques 32 γ sont insérés en tenant compte des intensités, textures et bruit classiques pour les organes et les images considérés.

### 6.1.4 Planification de traitement (14)

Les images ainsi simulées correspondant à des images de planification (images scanner) 131 sont intégrées dans un système commercial de planification de traitement (TPS : « Treatment Planning System » en anglais, par exemple le système Pinnacle de Philips (marques déposées)).

Un traitement de référence est planifié (14). Par exemple, pour le pelvis, on effectue une planification à une dose totale de 80 Gray dans la prostate, par une technique de RCMI (Radiothérapie conformationnelle avec modulation d'intensité). Cette phase d'optimisation de la balistique fournit la dose planifiée de référence 141 associée au scanner de planification et les paramètres de la balistique d'irradiation 142.

### 6.1.5 Calcul de la dose sur les images de suivi (15)

La dose 151 associée aux images de suivi (correspondant à la dose réellement reçue par les patients lors de la séance d'irradiation à laquelle l'image de suivi a été acquise), appelée doses par fraction, est calculée.

Deux options peuvent alors être envisagées suivant la localisation considérée, selon les cas :
(i) les contours externes du patient sont peu modifiés lors du traitement (cas du pelvis), la dose planifiée peut alors être reportée directement sur l'image de suivi;
(ii) les contours externes du patient sont modifiés (par exemple suite à la fonte tumorale en ORL), la dose doit alors être recalculée (par exemple sur un TPS) suivant la balistique d'irradiation planifiée.
Dans les deux cas, une translation de la dose peut être appliquée afin de prendre en compte le repositionnement du patient (par exemple un recalage rigide suivant la position de la prostate).

### 6.1.6 Calcul de la dose cumulée de référence (16)

Les champs de déformations 121 entre image de planification et images de suivi étant connus, ainsi que la dose planifiée 141 et/ou la dose associée aux images de suivi 151, la dose cumulée de référence 161 peut-être calculée (16).

Pour chaque image de suivi considérée, la dose associée est déformée suivant le champ de déformations simulé. La dose est ainsi propagée vers l'espace du scanner de référence et est donc cumulable dans cet espace.

Cette propagation est réalisée pour chaque image de suivi et l'ensemble des doses propagées sont sommées pour obtenir la dose cumulée de référence 161.

Plus précisément, le champ de déformations est composé d'un ensemble de vecteurs, défini en chaque noeud des maillages décrivant les organes dans leur configuration de référence. Ce champ de déformations met spatialement en correspondance la position de chaque noeud d'un maillage avec sa position après déformation. Ce champ de déformations, peu dense, peut être utilisé en l'état pour cumuler les doses sur les noeuds du maillage ou être interpolé afin de cumuler les doses en chaque point du volume. Si les coordonnées d'un point déformé ne correspondent pas à la grille d'échantillonnage de l'image de planification, la dose peut être interpolée.

### 6.2 Application du fantôme numérique

Les données du fantôme numérique simulées préalablement (images de planification 411 et de suivi (par exemple CBCT) 412, dose planifiée 413 et éventuellement doses par fraction 414) sont fournies (42) à l'outil évalué qui estime la dose cumulée 421 reçue par les organes, selon la méthode à évaluer.

Cette dose cumulée 421 estimée est comparée (43) à la dose cumulée de référence 161 déterminée selon l'invention, comme décrit précédemment, et des mesures de conformité sont générées.

Ces mesures peuvent être de différentes natures, en fonction des besoins, et comprendre notamment :
- une erreur d'estimation de la dose cumulée en chaque point du champ de déformations : représentation tridimensionnelle des erreurs ;
- des mesures statistiques sur les erreurs d'estimation de la dose cumulée (erreurs moyenne, minimale et maximale, écart-type, ...) ;
- une représentation des doses cumulées estimée et de référence sous la forme d'histogrammes dose-volume (HDV, mode de représentation de référence pour l'évaluation de la dose en routine clinique. Ceci correspond, pour un organe, à sa fraction de volume recevant au moins une dose donnée) ;
- des mesures statistiques issues des HDV (différences moyenne, minimale, maximale, somme des différences, ...) ;
- des différences en termes d'indices d'évaluation d'un traitement (dose moyenne, dose équivalente uniforme (EUD, « equivalent uniform dose » en anglais), valeurs prédictives de contrôle tumoral ou de toxicité (TCP, « tumor control probability » en anglais ; NTCP, « normal tissue complexity probability » en anglais), indices classiques d'évaluation des HDV.

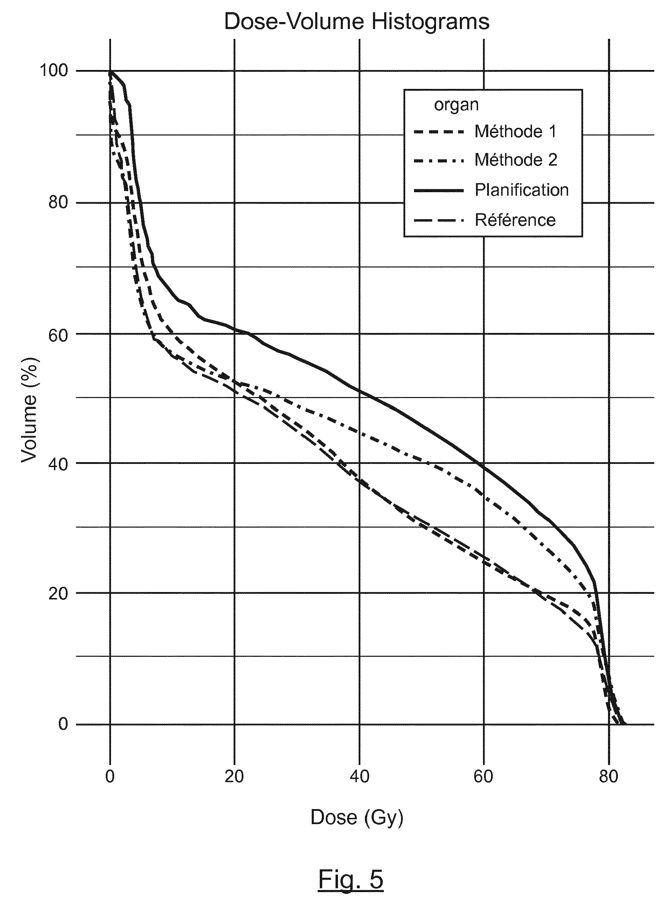

A titre d'exemple, la figure 5 illustre une comparaison d'histogrammes dose-volume calculés sur la vessie :
- HDV planifié 51,
- HDV cumulé de référence 52,
- HDV cumulé estimé par une première méthode commerciale 53,
- HDV cumulé estimé par une seconde méthode commerciale 54.

On constate que les résultats obtenus par la première méthode sont nettement supérieurs à ceux obtenus par seconde méthode. Ainsi, le procédé de l'invention permet de déterminer que cette première méthode est meilleure, alors qu'intuitivement, en se fiant à la courbe de planification 51, un observateur aurait pu imaginer de façon erronée que la seconde méthode, plus proche de la planification, était sans doute plus efficace.

### 6.3 Exemples d'applications

L'invention peut s'appliquer à de nombreux types de cancer, en particulier lorsqu'ils concernent des organes déformables. Outre le cancer de la prostate, illustré ci-dessus, elle peut par exemple être utilisée pour évaluer des méthodes de détermination de dose cumulée pour des cancers du col de l'utérus, de l'utérus, de la vessie, du rectum, ORL,...

## Revendications

1. Procédé d'évaluation d'une méthode de détermination d'une dose de radiothérapie cumulée reçue au cours d'un traitement de radiothérapie, dite méthode à évaluer, **caractérisé en ce que** :
- on définit un modèle déformable d'au moins un organe à traiter et d'au moins un organe voisin, et on génère à l'aide dudit modèle une série d'images simulées, interprétables par la méthode à évaluer;
- on compare la dose cumulée calculée par la méthode à évaluer, appliquée à au moins une desdites images simulées, à une dose cumulée de référence, de façon à délivrer au moins une information représentative de la qualité de ladite méthode,
ladite dose cumulée de référence étant obtenue en tenant compte de déformations anatomiques appliquées auxdits organes, de façon à déterminer une dose de référence effectivement reçue à chaque séance de traitement par lesdits organes, en fonction de leur forme et/ou de leur position réelles lors de cette séance.

2. Procédé d'évaluation selon la revendication 1, **caractérisé en ce que** l'obtention de ladite dose cumulée de référence met en oeuvre les étapes suivantes :
- génération d'un modèle comprenant au moins un organe à traiter et au moins un organe voisin ;
- détermination d'un champ de déformation desdits organes, à partir d'au moins une règle de déformation et/ou d'évolution desdits organes ;
- obtention d'une série d'images dudit modèle à différents instants, dites images simulées, comprenant au moins une image représentative d'un scanner de planification et une série d'images de suivi, correspondant chacune à une séance de traitement ;
- planification d'un traitement, à partir d'au moins une desdites images simulées, à l'aide d'un système de planification de traitement mettant en oeuvre ladite méthode de détermination de doses de radiothérapie, de façon à obtenir une dose planifiée et des paramètres d'une balistique d'irradiation ;
- détermination d'un ensemble de doses par fraction, correspondant chacune à une desdites séances ;
- détermination de ladite dose cumulée de référence, en appliquant ledit champ de déformation à chaque image de suivi, de façon à déterminer ladite dose de référence effectivement reçue.

3. Procédé d'évaluation selon la revendication 2, **caractérisé en ce que** ladite étape de génération d'un modèle délivre un modèle en éléments finis sous la forme d'un maillage en trois dimensions.

4. Procédé d'évaluation selon l'une quelconque des revendications 2 et 3, **caractérisé en ce que** ladite étape de détermination d'un champ de déformation met en oeuvre des déformations représentatives de l'élasticité de chaque organe, définies par une loi de comportement de matériaux, par exemple un modèle hyper-élastique.

5. Procédé d'évaluation selon l'une quelconque des revendications 2 à 4, **caractérisé en ce que** lesdites déformations tiennent compte des contacts possibles entre lesdits organes.

6. Procédé d'évaluation selon l'une quelconque des revendications 2 à 5, **caractérisé en ce que** ladite étape d'obtention d'une série d'images simulées associe, pour chaque image simulée, une vue dudit modèle et une image, éventuellement simplifiée, d'un patient réel, afin de prendre en compte au moins la structure osseuse de ce dernier.

7. Procédé d'évaluation selon l'une quelconque des revendications 2 à 6, **caractérisé en ce que**, lors de ladite étape de détermination de ladite dose cumulée de référence, la dose associée à chaque image de suivi considérée est déformée suivant le champ de déformations simulé, de façon à propager ladite dose vers l'espace du scanner de référence, et l'ensemble des doses propagées sont sommées pour obtenir la dose cumulée de référence.

8. Procédé d'évaluation selon l'une quelconque des revendications 1 à 7, **caractérisé en ce que** la ou lesdites informations représentatives de la qualité de ladite méthode à évaluer appartiennent au groupe comprenant :
- une erreur d'estimation de la dose cumulée en chaque point du champ de déformations, par exemple sous la forme d'une représentation tridimensionnelle des erreurs ;
- des mesures statistiques sur les erreurs d'estimation de la dose cumulée, telles que des erreurs moyenne, minimale et maximale, des écart-type ;
- une représentation des doses cumulées estimée et de référence sous la forme d'histogrammes dose-volume ;
- des mesures statistiques issues d'histogrammes dose-volume, telles que des différences moyenne, minimale, maximale, des sommes des différences ;
- des différences en termes d'indices d'évaluation d'un traitement, telles qu'une dose moyenne, une dose équivalente uniforme, des valeurs prédictives de contrôle tumoral ou de toxicité.

9. Procédé d'évaluation selon l'une quelconque des revendications 1 à 8, **caractérisé en ce que** ledit organe à traiter appartient au groupe comprenant la prostate, le rectum, la vessie et le canal anal.

10. Produit programme d'ordinateur téléchargeable depuis un réseau de communication et/ou stocké sur un support lisible par ordinateur et/ou exécutable par un microprocesseur, **caractérisé en ce qu'**il comprend des instructions de code de programme pour l'exécution du procédé d'évaluation selon l'une au moins des revendications 1 à 8, lorsqu'il est exécuté sur un ordinateur.

## Patentansprüche

1. Auswertungsverfahren einer Methode zum Bestimmen einer kumulierten während einer Strahlentherapie erhaltenen Strahlendosis, genannt auszuwertende Methode, **gekennzeichnet dadurch, dass** das Verfahren die folgenden Schritte aufweist:
- Man bestimmt ein verformbares Modell zumindest eines zu behandelnden Organs und zumindest eines benachbarten Organs und anhand des Modells generiert man eine Reihe simulierter Bilder, die durch die auszuwertende Methode interpretierbar sind;
- Man vergleicht die mittels der auszuwertenden Methode, die auf zumindest eines der simulierten Bilder angewandt wurde, errechnete kumulierte Dosis mit einer kumulierten Referenzdosis, um zumindest eine für die Qualität der Methode repräsentative Information zu liefern,
wobei die kumulierte Referenzdosis unter Berücksichtigung von auf die Organe angewandten anatomischen Verformungen ermittelt wird, um eine von den Organen bei jeder Behandlungssitzung tatsächlich erhaltene Referenzdosis als eine Funktion ihrer Form und/oder ihrer tatsächlichen Lage während dieser Sitzung.

2. Auswertungsverfahren nach Anspruch 1, **dadurch gekennzeichnet, dass** der Erhalt der kumulierten Referenzdosis die folgenden Schritte implementiert:
- Erzeugen eines Modells mit mindestens einem zu behandelnden Organ und mindestens einem benachbarten Organ;
- Bestimmen eines Verformungsfeldes der Organe auf Basis von mindestens einer Verformungs- und/oder Entwicklungsregel der Organe;
- Erhalten einer Reihe von Bildern des Modells zu unterschiedlichen Zeitpunkten, genannt simulierte Bilder, mit mindestens einem repräsentativen Bild eines Planungsscanners und einer Reihe von Folgebildern, wovon jedes einer Behandlungssitzung zuzuordnen ist;
- Planen einer Behandlung auf Basis von mindestens einem der simulierten Bilder anhand eines Behandlungsplanungssystems unter Verwendung der Methode zum Bestimmen von Strahlensdosen, um eine geplante Dosis und ballistische Bestrahlungsparameter zu erhalten;
- Bestimmen eines Satzes von anteiligen Dosen, wobei jede einer der Sitzungen entspricht;
- Bestimmen der kumulierten Referenzdosis durch Anwenden des Verformungsfeldes auf jedes Folgebild, um die tatsächlich erhaltene Referenzdosis zu bestimmen.

3. Auswertungsverfahren nach Anspruch 2, **dadurch gekennzeichnet, dass** der Schritt des Erzeugens eines Modells ein Modell mit finiten Elementen in Form dreidimensionaler Maschen liefert.

4. Auswertungsverfahren nach einem der Ansprüche 2 oder 3, **dadurch gekennzeichnet, dass** der Schritt des Bestimmens eines Verformungsfelds Verformungen implementiert, die für Elastizität jedes Organs repräsentativ sind und durch ein Gesetz des Materialverhaltens definiert werden, beispielsweise ein hyperelastisches Modell.

5. Auswertungsverfahren nach einem der Ansprüche 2 bis 4, **dadurch gekennzeichnet, dass** die Verformungen die möglichen Berührungen der Organe untereinander berücksichtigen.

6. Auswertungsverfahren nach einem der Ansprüche 2 bis 5, **dadurch gekennzeichnet, dass** der Schritt des Erhaltens einer Reihe von simulierten Bildern für jedes simulierte Bild eine Ansicht des Modells und ein Bild, gegebenenfalls vereinfacht, eines tatsächlichen Patienten zuordnet, damit zumindest die Knochenstruktur dieses Patienten berücksichtigt werden kann.

7. Auswertungsverfahren nach einem der Ansprüche 2 bis 6, **dadurch gekennzeichnet, dass**, bei dem Schritt des Bestimmens der kumulierten Referenzdosis, die jedem berücksichtigten Folgebild zugeordnete Dosis entsprechend dem simuliertem Verformungsfeld verformt wird, um die Dosis in den Raum des Referenz-Scanners zu propagieren, wobei die Summe der propagierten Dosen die kumulierte Referenzdosis ergibt.

8. Auswertungsverfahren nach einem der Ansprüche 1 bis 7, **dadurch gekennzeichnet, dass** die für die Qualität der auszuwertenden Methode repräsentative(n) Information(en) zu der Gruppe gehört oder gehören umfassend:
- Ein Fehler bei der Schätzung der kumulierten Dosis an jedem Punkt des Verformungsfeldes, zum Beispiel in Form einer dreidimensionalen Darstellung der Fehler;
- Statistische Messungen der Fehler bei der Schätzung der kumulierten Dosis, wie Mittelwert, Minimum und Maximum, Standardabweichung;
- Eine Darstellung der geschätzten kumulierten Dosen und der Referenzdosen in Form eines Dosis/Volumen-Histogramms;
- Statistische Messungen aus dem Dosis/Volumen-Histogramm, wie durchschnittliche, minimale, maximale Unterschiede und Summen der Unterschiede;
- Unterschiede in Bezug auf die Auswertungsindizes einer Behandlung, wie beispielsweise eine durchschnittliche Dosis, eine gleichmäßige Äquivalenzdosis, Vorhersagewerte für die Kontrolle von Tumoren oder der Toxizität.

9. Auswertungsverfahren nach einem der Ansprüche 1 bis 8,
**dadurch gekennzeichnet, dass** das zu behandelnde Organ zu der Gruppe umfassend die Prostata, das Rektum, die Blase und den Analkanal gehört.

10. Computerprogrammprodukt, das aus einem Kommunikationsnetz herunterladbar und/oder auf einem computerlesbaren Medium gespeichert ist und/oder von einem Mikroprozessor ausführbar ist, **dadurch gekennzeichnet, dass** es Programmcodeanweisungen zum Ausführen des Auswertungsverfahrens nach mindestens einem der Ansprüche 1 bis 8 aufweist, wenn sie auf einem Computer ausgeführt werden.

## Claims

1. Evaluation method for a method for determining an accumulated dose of radiotherapy received during a radiotherapy treatment, known as the method to evaluate, **characterised in that**:
- a deformable model is defined, of at least one organ to treat and at least one nearby organ, and a series of simulated images are generated, using the said model, interpretable by the method to evaluate;
- the accumulated dose calculated by the method to evaluate is compared, applied to a least one of the said simulated images, to an accumulated reference dose, in a way that at least one piece of information representing the quality of the said method is delivered,
the said accumulated reference dose being obtained by taking into account anatomic deformations actually received at each treatment session by the aid organs, according to their actual shape and/or their actual position during this session.

2. Evaluation method according to Claim 1, **characterised in that** obtaining the said accumulated reference dose implements the following steps:
- generating a model, including at least one organ to treat and at least one nearby organ;
- determining a deformation field of the said organs, from at least one rule of deformation and/or development of the said organs;
- obtaining a series of images from the said model at different times, known as simulated images, including at least one image representative of a planning scanner and a series of follow-up images, each one corresponding to a treatment session;
- planning a treatment, from at least one of the said simulated images, using a treatment planning system, implementing the said method for determining radiotherapy doses, in a way to obtain a planned dose and the parameters of irradiation ballistics;
- determining a set of fraction doses, each one corresponding to one of the said sessions;
- determining the said accumulated reference dose, by applying the said deformation field to each follow-up image, in a way to determine the said reference dose actually received.

3. Evaluation method according to Claim 2, **characterised in that** the said step for generating a model delivers a model of finished elements, in the form of a three-dimensional mesh.

4. Evaluation method according to any one of Claims 2 and 3, **characterised in that** the said step for determining a deformation field implements deformations representative of the elasticity of each organ, defined by a material behaviour law, for example, a hyper-elastic model.

5. Evaluation method according to any one of Claims 2 to 4, **characterised in that** the said deformations take into account possible contact between the said organs.

6. Evaluation method according to any one of Claims 2 to 5, **characterised in that** the said step for obtaining a series of simulated images, associates, for each simulated image, a view of the said model and an image, possibly simplified, of a real patient, in order to take into account at least the patient's bone structure.

7. Evaluation method according to any one of Claims 2 to 6, **characterised in that**, during the said step for determining the said accumulated reference dose, the dose associated with each follow-up image taken into account is deformed according to the simulated deformations field, in a way to extend the said dose to the reference scanner space, and all extended doses are added up to obtain the accumulated reference dose.

8. Evaluation method according to any one of Claims 1 to 7, **characterised in that** the said information representative of the quality of the said method to evaluate, belongs to the group, including:
- an error in estimating the accumulated dose at each point in the deformations field, for example, in the form of a three-dimensional representation of the errors;
- statistical measurements on errors in estimating the accumulate dose, such as average, minimum and maximum errors, standard deviations;
- an estimated and reference representation of accumulated doses, in the form of dose/volume histograms;
- statistical measurements from dose/volume histograms, such as average, minimum, maximum differences, sums of differences;
- difference in terms of treatment evaluation indices, such as an average dose, an equal uniform dose, tumour or toxicity test predicted values.

9. Evaluation method according to any one of Claims 1 to 8, **characterised in that** the said organ to be treated belongs to the group including the prostate, the rectum, the bladder and the anal canal.

10. Computer programme product, downloadable from a communication network and/or stored on a piece of media, which can be read by a computer and/or can be executed by a microprocessor, **characterised in that** it includes programme code instructions to execute the method to evaluate, according to at least one of Claims 1 to 8, when it is executed on a computer.
